# FASCICULE DE BREVET EUROPEEN

(11) **EP 3 439 574 B1**
(45) Date de publication et mention de la délivrance du brevet: **13.05.2020**
(21) Numéro de dépôt: 17720547.3
(22) Date de dépôt: 05.04.2017
(51) Int. Cl.: A61C 7/10

(54) **DISPOSITIF D'EXPANSION PALATINE**
GAUMENDEHNUNGSVORRICHTUNG
PALATAL EXPANSION DEVICE

(30) Priorité: 08.04.2016 FR 1653141
(43) Date de publication de la demande: 13.02.2019
(73) Titulaire: Atlan, David, 95130 Franconville-La-Garenne (FR)
(72) Inventeur: Atlan, David, 95130 Franconville-La-Garenne (FR)
(74) Mandataire: Lefevre-Groboillot, David André
(86) Numéro de dépôt international: PCT/FR2017/050801
(87) Numéro de publication internationale: WO 2017/174929

(56) Documents cités:
- DE-A1- 10 234 376
- FR-A1- 2 742 039
- KR-A- 20090 120 895
- US-A1- 2002 018 978
- US-B1- 6 358 255
- US-B1- 6 358 255

## Description

### Contexte technique

L'invention s'inscrit dans le domaine de l'orthodontie, de l'orthopédie dento faciale (odf) et de la chirurgie maxillo-faciale, notamment humaines.

On connaît des dispositifs d'expansion palatine, ou disjoncteur maxillaire, utilisés pour élargir le palais et la mâchoire supérieure, chez les enfants, les adolescents, ou dans certains cas les adultes. Ils sont utilisés quand la mâchoire supérieure est trop étroite. Ils permettent de faire grandir l'ensemble constitué des deux os maxillaires dans le sens transversal, ainsi que d'élargir les sutures palatines médianes et les sutures incisives. On recherche un élargissement ou une disjonction des sutures par un effet squelettique et un élargissement de l'arcade dentaire par effet alvéolo-dentaire, selon le type d'appui, de pression et l'âge du patient.

Dans ces dispositifs, un vérin central est activé sur prescription afin d'effectuer la distraction ou disjonction osseuse centripète. Le vérin peut être intégré dans une plaque amovible, ou collé sur les dents par le moyen de gouttières thermoformées ou de bagues. Le système utilise des appuis osseux sur les os maxillaires, ou dentaires sur les molaires, les prémolaires et les canines.

L'ensemble du dispositif est fabriqué sur mesure par un laboratoire de prothèses.

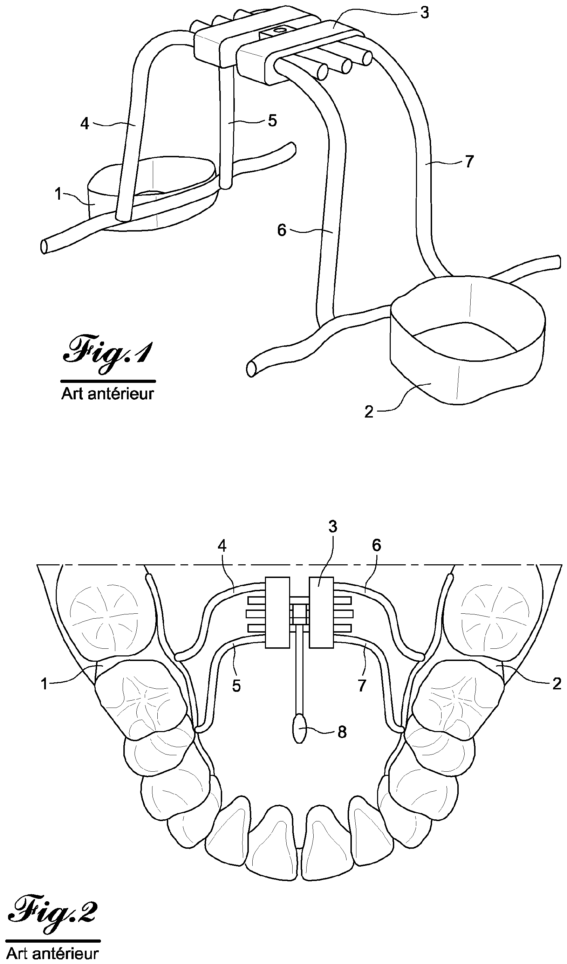

En figure 1, on a représenté un tel dispositif d'expansion palatine connu, présenté à titre d'exemple. Il comprend des pièces d'appui 1 et 2, constituées ici chacune d'une bague qui enserre une molaire de chaque côté du palais et prend appui sur la molaire enserrée et les dents voisines par l'intermédiaire de barres d'extensions mésiales ou distales d'appui. Il comprend aussi un vérin central 3. Le vérin central 3 est réglable pour assurer un écartement transversal entre les deux bagues 1 et 2. Des tiges rigides 4, 5 et 6, 7 assurent la liaison fixe entre le vérin central 3 et les pièces d'appui 1 et 2, en permettant au dispositif d'être aussi peu gênant et discret que possible. Ces tiges assurent les liaisons entre le vérin central et les dents, mais assurent aussi la rigidité, la transmission et la répartition des forces nécessaires aux déplacements voulus. Des matériaux comme l'acier inoxydable ou le titane peuvent être utilisés, ainsi que d'autres matériaux biocompatibles y compris des alliages biocompatibles. En figure 2, on a représenté le dispositif mis en place dans la bouche d'un patient, qui le porte pendant plusieurs semaines ou plusieurs mois. Au fur et à mesure que le dispositif produit ses effets, le vérin est réglé (ou « activé ») pour maintenir un effort d'écartement, augmentant ainsi progressivement la distance entre les deux parties de la mâchoire et/ou les différents os. Le réglage se fait en tournant une vis centrale du vérin qui provoque l'expansion transverse. La vis est tournée avec un outil adapté, telle une clé 8. Quand l'expansion souhaitée est atteinte, l'appareil est maintenu en place pendant une certaine période pour consolider le traitement, puis il est retiré.

EP09190207 divulgue un dispositif de disjonction maxillaire, utilisant un élément de réception concave pour chaque bout de bras associé à une surface de fixation pour association à la dent correspondante, l'élément de réception étant couplé au bout de bras par interférence ou rétrécissement mécanique ou soudure par point, ou soudure laser, ou moyens d'adhésion.

US20130252195 divulgue un dispositif de disjonction maxillaire en kit avec utilisation de bras de différentes géométries à choisir.

US 2002/0018978 A1 divulgue un dispositif d'expansion palatale dont les fixations se font directement sur l'os.

Malgré les dispositifs existants, on ne dispose pas actuellement de solution réellement flexible, permettant une mise en place rapide sans passer par un prothésiste, et qui soit adaptable, par une présentation en kit à une mise en place sur chaque patient sans nécessiter une prise d'empreinte et un long parcours par un cabinet de prothésiste.

### Définition de l'invention et avantages associés

Pour résoudre les problèmes ainsi identifiés, il est proposé un dispositif d'orthodontie selon les revendications 1 à 8.

Un tel système offre une grande modulabilité, peut être préfabriqué, et est facilement ajustable et adaptable pour chaque patient. Il est ajustable, modulable en bouche au fauteuil ou hors bouche, et il est fixable, c'est-à-dire qu'il est possible ensuite de figer sa position. Le système dispose d'une articulation qui existe uniquement pour l'ajustement et qui est ensuite contrariée une fois finie la mise en place.

Selon différentes caractéristiques avantageuses,
- l'ajustement est possible dans le sens vertical, le sens antéro-postérieur et le sens transversal ;
- au moins une des poutres est télescopique ou à tout le moins ajustable en longueur pour permettre un ajustement géométrique pendant une mise en place du dispositif dans la bouche du patient ;
- au moins une des poutres est démontable, le dispositif comprenant au moins une poutre de remplacement d'une longueur différente pour permettre un ajustement géométrique pendant une mise en place du dispositif dans la bouche du patient ;
- le dispositif comprend une liaison mécanique en rotation à l'extrémité de chaque poutre entre la poutre et le vérin ;
- il comprend une liaison mécanique en rotation à l'extrémité de chaque poutre opposée au vérin, à interposer entre la poutre et la mâchoire ;
- il comprend des attaches à coller sur les dents, par scellement ou collage ou rétention mécanique ou chimique, et portant des moyens de liaison pour relier les attaches au vérin, le dispositif comprenant de plus des éléments complémentaires de liaison reliés au vérin pour mettre en place le dispositif dans la bouche du patient à l'aide des attaches ;
- il comprend des attaches de différentes dimensions et formes pour l'adaptation à différents types de dents et de morphologies ;
- au moins une des attaches comprend une zone de fragilité ou de faiblesse permettant un enlèvement par déformation ;
- les attaches dont il existe plusieurs types en fonction de l'importance de la pression et du type de mouvement à appliquer portent des encoches simples externes, des doubles encoches externes, des doubles encoches internes externes ou une boule externe pour une liaison par emboîtement, ou des éléments de vissage pour une liaison par vissage ;
- le vérin est fixé par encastrement ou tout autre moyen de fixation sur un support gauche et un support droit libres l'un par rapport à l'autre.

Grâce à ces différentes caractéristiques, on dispose d'un vérin de disjonction équipé de bras articulés dans les trois dimensions. L'ensemble est proposé en kit, avec des pièces ajustables de différentes longueurs. Aux extrémités de chaque bras, un système de fixation permet de fixer les bras sur les dents, en s'adaptant à chaque dent, et en s'adaptant à la distance et à l'orientation entre le vérin central et la dent. L'ensemble du système permet la mise en place du dispositif par un collage fin et adaptable où tous les éléments son préfabriqués et ajustés directement dans la bouche du patient. Il en résulte un gain de temps, et un confort pour le praticien et le patient, ainsi qu'une meilleure précision. Une méthode de pose d'un dispositif d'orthodontie est également décrite, celle-ci comprenant une étape de formation d'un premier appui pour le dispositif sur la gauche d'une mâchoire d'un patient à soigner, une étape de formation d'un deuxième appui pour le dispositif sur la droite de ladite mâchoire supérieure, et une étape de modulation de l'écartement entre les deux mâchoires par un vérin central du dispositif, **caractérisée en ce que** la méthode de pose comprend de plus une étape d'ajustement d'un angle entre le vérin et ledit premier appui ou d'un angle entre le vérin et ledit deuxième appui pour permettre un ajustement géométrique du dispositif dans la bouche du patient ou hors de la bouche adapté à la morphologie de celle-ci, au moins dans le sens vertical, le sens antéro-postérieur ou le sens transversal.

Les étapes de formation des appuis peuvent comprendre la mise en place d'un support sur la mâchoire, et/ou la disposition du dispositif en appui sur la mâchoire.

La description va se poursuivre en lien avec les figures, présentées à titre d'illustration.

### Liste des figures

La figure 1 présente un système selon l'art antérieur.
La figure 2 présente le système de la figure 1 en position.
La figure 3 présente une vue générale d'un système selon l'invention, au cours de sa mise en place.
La figure 4 présente une vue générale du système de la figure 3, une fois fixé.
Les figures 5a à 5d présentent un premier aspect de l'invention, à savoir des attaches à placer sur les dents, dans 4 variantes différentes.
Les figures 6a à 6d présentent un détail de l'aspect de l'invention précédent, dans différentes variantes.
La figure 7 présente une variante particulière de l'aspect précédent.
Les figures 8a et 8b présentent une autre variante particulière de l'aspect précédent.
La figure 9 présente une vue générale de la mise en place d'un système selon l'invention montrant l'aspect précédent.
Les figures 10a à 10c présentent un deuxième aspect de l'invention, constitué d'un vérin portant des rotules.
Les figures 11a et 11b montrent une première propriété importante d'un système selon l'invention. La figure 11c présente une variante de mise en œuvre. La figure 11d présente une option.
Les figures 12a à 12c montrent une deuxième propriété importante d'un système selon l'invention, en liaison avec un quatrième aspect de l'invention, constitué d'une poutre télescopique.
Les figures 13a à 13c montrent une troisième propriété importante d'un système selon l'invention, en liaison avec un cinquième aspect de l'invention, constitué d'un élément de fixation en rotation vis-à-vis d'une poutre.
La figure 14 montre un sixième aspect de l'invention, constitué d'une fixation par clipsage dent par dent.
La figure 15 montre le même aspect, dans une variante.
Les figures 16a à 16c montrent trois réglages possibles du système selon l'invention, rendus possibles grâce aux différents aspects discutés.
La figure 17 montre une option possible pour le système selon l'invention.
La figure 18 montre une autre option possible pour le système selon l'invention.
La figure 19 montre encore une autre option possible pour le système selon l'invention.

### Description détaillée

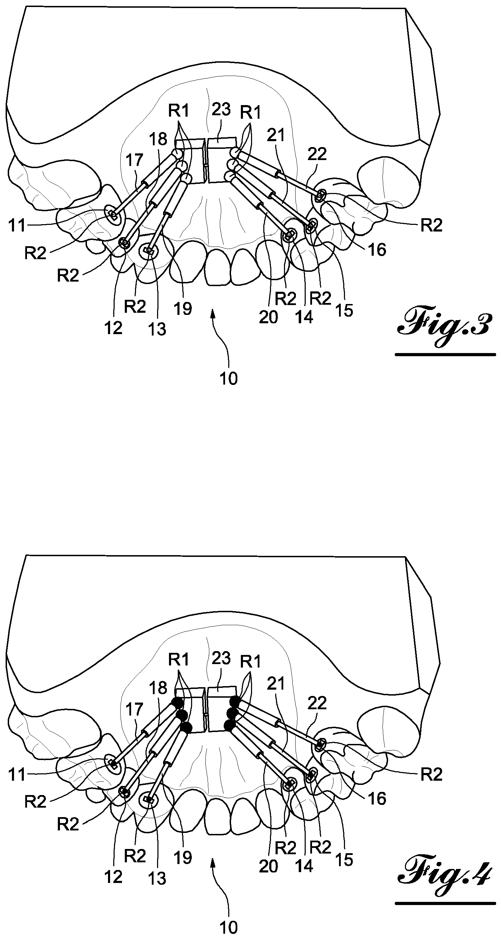

En **figure 3****,** on a représenté un système d'expansion palatine 10 selon l'invention, en place au niveau de la mâchoire supérieure d'un patient. Le système d'expansion palatine 10 comprend six éléments de fixation aux dents 11 à 16, dont trois, référencés 11 à 13, sur les dents d'un côté de la mâchoire et trois autres, référencés 14 à 16, sur les dents correspondantes de l'autre côté de la mâchoire. La fixation et l'appui peuvent être dentaires, gingivaux et/ou osseux.

Le système d'expansion palatine comprend de plus six bras d'appui (ou poutres) 17 à 22 s'appuyant chacun sur un des éléments de fixation 11 à 16. Il comprend de plus un vérin central 23. Les bras d'appui 17 à 22 relient chacun un des éléments de fixation 11 à 16, respectivement au vérin central 23. Les bras d'appui 17 à 22 sont tous des bras télescopiques, ou du moins des bras ajustables dans leur longueur par l'un ou l'autre de plusieurs systèmes possibles : vérin pneumatique, bras télescopique, pas de vis, bras cranté..., pouvant adapter diverses longueurs, dont les valeurs peuvent être choisies de manière continue ou discrète.

Chacun des bras d'appui est relié au vérin central par une rotule R1 associée, et à l'élément de fixation aux dents correspondant par une deuxième rotule R2 associée.

En figure 3, le système est en phase de mise en place, et les 12 rotules R1 ou R2 sont partiellement ou totalement libres et peuvent donc permettre des mouvements entre le vérin, les bras et les dents.

En **figure 4****,** on a représenté le système d'expansion palatine 10 après sa rigidification. Un ou plusieurs éléments de rigidification rigidifient les six rotules R1 liant le vérin central aux bras. Des éléments de rigidification sont également mis en place au niveau des rotules R2 reliant les bras et les éléments de fixation aux dents 11 à 16. Les éléments de rigidification peuvent être tous de même nature, ou être différents. Différentes stratégies de rigidification peuvent être utilisées. Elles peuvent utiliser un élément de rigidification intégré au système d'expansion palatine 10, par exemple intégré aux rotules R1 et R2, ou amené de l'extérieur, tel de la colle, comme une colle composite, un ciment verre ionomère, un produit d'obturation IRM (marque de Dentsply) ou une résine à activation par photopolymérisation ou chémopolymérisation.

Dans un mode de réalisation, une fois le système verrouillé, il peut être déverrouillé avec une clé de déverrouillage ou tout outil externe. Dans d'autres modes de réalisation, une fois le système verrouillé, il ne peut pas être déverrouillé, et doit donc, pour être défait, être dans le cas d'une photopolymérisation pour défaire il faut casser. C'est le cas par exemple dans le cas d'un verrouillage par photopolymérisation.

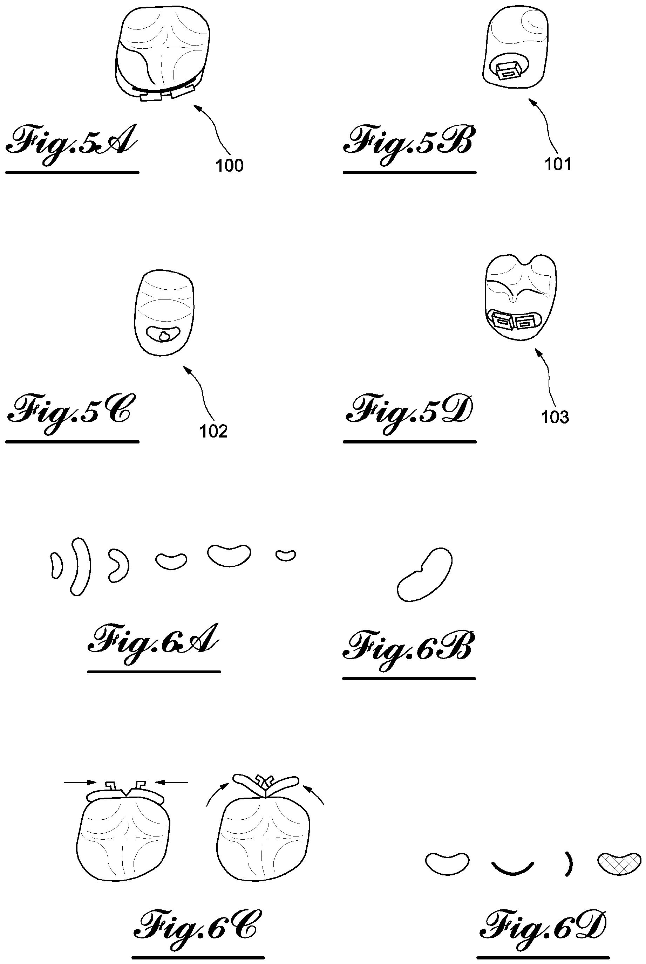

En **figure 5****,** on a représenté différents éléments de fixation pouvant être utilisés pour fixer les bras aux dents. La fixation est effectuée par connexion, notamment par clipsage, d'un élément présent à l'extrémité du bras, et d'un élément pré-positionné sur la surface latérale de la dent du côté de l'intérieur de la bouche (face palatine ou linguale de la dent). Ces deux éléments à clipser sont complémentaires et peuvent avoir différentes formes, qui peuvent par exemple être qualifiées de formes mâle et femelle. Dans le cas d'un couple de formes mâle et femelle, la forme mâle peut indifféremment être sur le bras, ou fixé à la dent. Le clipsage implique une forme de verrouillage.

En figure 5a, la pièce fixée à la dent présente deux formes disjointes séparées par un espace, et est qualifiée de fixation à double encoche mâle 100. Chacune des formes présente en effet un espace pour engager une forme complémentaire attachée au bras. Cette forme complémentaire est alors engagée entre la pièce et la dent. Les deux formes disjointes sont disposées dos à dos, ce qui justifie l'appellation « interne/externe ». Les espaces d'engagement permettent conjointement de proposer un système de retenu de la pièce complémentaire attachée au bras.

En figure 5b, la pièce fixée à la dent présente une unique fente et est qualifiée d'attache simple encoche femelle 101.

En figure 5c, la pièce fixée à la dent présente une boule externe et est qualifiée d'attache à boule mâle 102.

En figure 5d, la pièce fixée à la dent présente une double encoche externe et est qualifiée d'attache à double encoche femelle 103.

En **figure 6****,** on a représenté la surface de collage des éléments de fixation aux dents discutés précédemment. Ces attaches sont de différentes tailles, courbures et formes pour s'adapter aux différentes morphologies dentaires : petite taille pour fixation sur une incisive ou une canine, moyenne pour une fixation à une prémolaire, ou plus importante pour une fixation sur une molaire, comme cela est représenté en figure 6a. En figure 6b, on a représenté un mode de réalisation dans lequel la surface de collage présente une zone de fragilité pour permettre un retrait aisé par déformation. Les efforts à effectuer pour déformer l'attache mise en place sont présentés dans la partie gauche de la figure 6c, et le résultat sur l'attache, vue de profil, est présenté dans la partie droite de cette figure 6c : les deux parties de l'attache entourant la partie fragile se déforment, libérant alors la dent.

En figure 6d, on a représenté une surface de collage de taille moyenne sous différents angles : de face, vue de dessus, vue de profil et vue de derrière. L'élément est une pièce mince, courbée pour épouser une surface de dent, et présentant une face interne grillagée pour permettre la rétention mécanique de la colle. Il y a alors un collage sur la surface de la dent et une rétention mécanique sur la surface de l'attache.

De manière générale, le collage se fait au niveau de la dent ou de la prothèse dentaire par un collage chimique, et du côté de l'attache par un collage chimique, si le revêtement est approprié, ou une rétention mécanique par un grillage ou une surface de rétention adaptée.

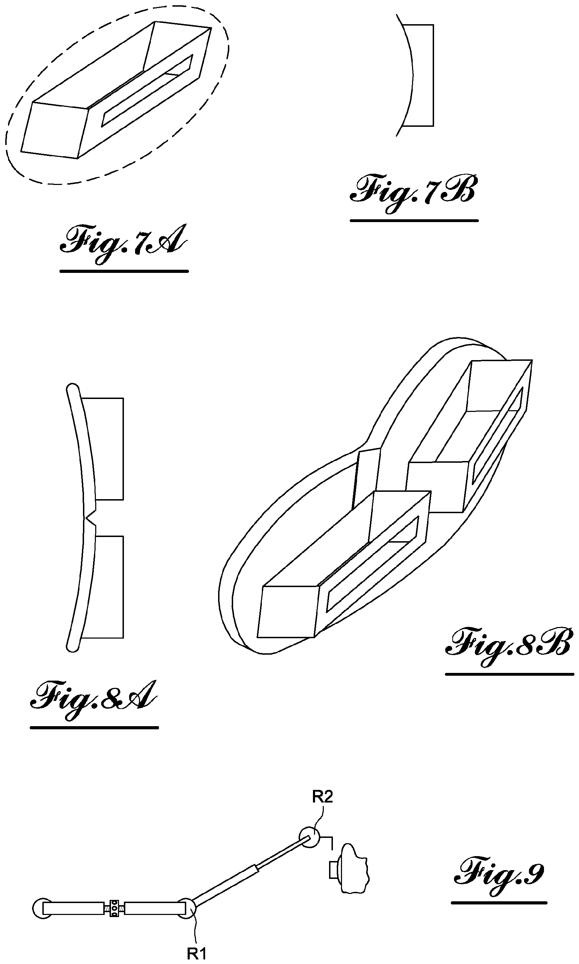

En **figure 7****,** on a représenté le détail de l'élément de fixation de type attache simple encoche externe. Il est composé d'un élément qui peut être sommairement parallélépipédique suffisamment large pour pouvoir limiter toute mobilité de l'axe de la poutre et de la poutre elle-même, en rotation. L'attache présente sur sa face opposée à la surface de collage une encoche de fixation.

En **figure 8****,** on a représenté le détail d'un élément de fixation de type attache double encoche. En figure 8a, l'élément est vu du dessus, alors qu'en figure 8b, il est vu de côté (vue latéro-supérieure). On a représenté la surface de collage à la dent dans sa version présentant une faiblesse pour faciliter le décollement. Les deux parties de l'élément de fixation de type attache double encoche sont placées de part et d'autre de la zone de faiblesse. En exerçant avec une pince une pression de chaque côté de la zone de faiblesse, on engendre la déformation de la surface de collage et son décollement.

En **figure 9****,** on a représenté la mise en place d'un bras dans la bouche d'un patient, le vérin étant maintenu temporairement près du palais. Un élément de fixation présent à l'extrémité du bras vient interagir avec un élément de fixation complémentaire collé à la dent, pour assurer une forme de clipsage entre le bras et la dent. Les rotules R1 et R2 aux deux extrémités du bras permettent à l'ensemble des éléments d'être positionnés avec flexibilité pour un ajustement total du dispositif dans la bouche du patient.

L'opération est répétée pour les différents bras.

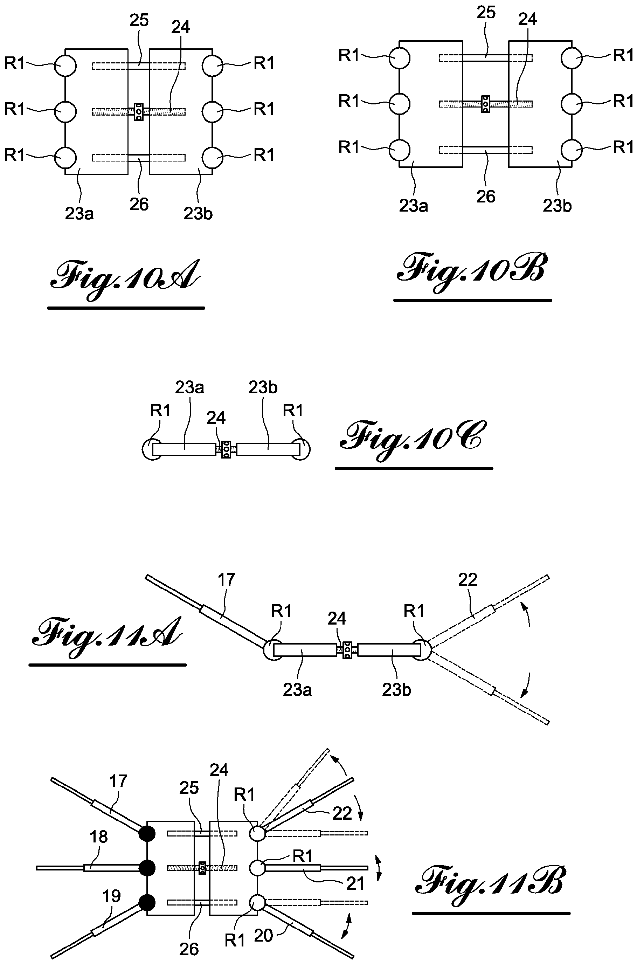

En **figure 10****,** on a représenté le vérin central 23, aussi appelé base. Il comprend deux éléments identiques qui sont des parallélépipèdes rectangles, ou demi-bases 23a et 23b se faisant face symétriquement par rapport à un plan P de symétrie générale du système. Le plan P de symétrie générale du système a vocation à être placé, dans certaines situations globalement sur le plan de symétrie générale de la bouche du patient, ou au contraire dans d'autres situations de manière désaxée, pour obtenir une application asymétrique des forces de part et d'autre de la mâchoire. Le plan P est positionné selon la morphologie et la dysmorphose du patient, et pour exercer les forces nécessaires à la résolution de la dysmorphose.

Les deux demi-bases 23a et 23b sont séparées par une vis 24 opérable par une clé, qui les écarte ou au contraire les rapproche l'un de l'autre de part et d'autre du plan de symétrie. La vis 24 est entourée de deux arbres 25 et 26 qui permettent de maintenir l'ensemble constitué des deux demi-bases 23a et 23b rigide.

Les figures 10a et 10b montrent le vérin en vue de dessus ou du dessous, alors que la figure 10c montre le vérin en vue de face. La figure 10a montre le vérin 23 en position assez fermée, alors que la figure 10b montre, sous le même angle, le vérin 23 en position plutôt ouverte. Sur les différentes vues, on voit les six rotules R1, dont trois sont attachées à la première demi-base 23a, et trois autres, symétriques des trois premières par rapport au plan P, sont attachées à la deuxième demi-base 23b. Ces six rotules R1 permettent de lier le vérin aux six bras (non représentés).

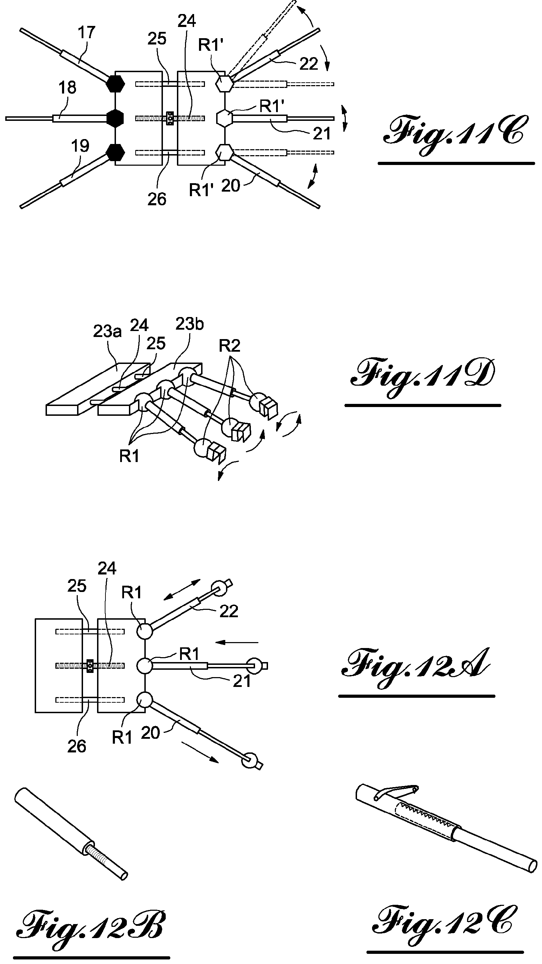

Les **figures 11a** à **11d** montrent les degrés de liberté des bras 17 à 22 autour de la base dans les directions verticale et antéro-postérieure. La figure 11a montre l'ajustement vertical, alors que les figures 11b et 11c montrent l'ajustement antéro-postérieur.

En figure 11a, on a représenté la base vue de face. La figure est dans un plan parallèle à un plan comprenant deux rotules R1 reliant la base et les deux bras 18 et 21, et étant parallèle à la vis 24, mais perpendiculaire au plan formé par la vis 24 et les arbres 25 et 26. On a représenté une rotation d'un bras autour d'un axe perpendiculaire au plan de la figure. Il s'agit ici du bras 21, représenté coplanaire avec la vis 24.

En figure 11b, qui représente la base vue de dessus, la figure étant dans un plan parallèle au plan formé par la vis 24 et les arbres 25 et 26, on a représenté des rotations de bras autour d'un axe perpendiculaire au plan de la figure. Il s'agit ici des bras 20, 21 et 22 représentés coplanaires avec la vis 24 et les arbres 25 et 26.

Le troisième degré de liberté en rotation autour de la rotule R1, qui correspond à une rotation autour de l'axe du bras 17 à 22, peut être présent ou absent (figure 11d). Les liaisons mécaniques en rotation R1 sont ainsi choisies parmi les liaisons rotule complète (trois degrés de rotation), rotule à doigt (deux degrés de rotation, visualisés aux figures 11a à 11c), voire dans une version plus simple, pivot (un seul degré de rotation, choisi parmi les deux degrés de rotation présentés aux figures 11a à 11c).

Les rotations présentées ci-dessus peuvent être continues.

En figure 11c, on a présenté une version dans laquelle les rotules R1 sont remplacées par des liaisons R1' permettant un ajustement discret d'un angle. Cela peut se faire par un système à formes complémentaires polygonales et régulières à encastrer. L'exemple représenté est hexagonal. Le système peut être utilisé sur un, deux ou trois degrés de liberté en rotation, auquel cas il utilise un encastrement de formes polyédriques.

En **figure 12 (12a, 12b et 12c****),** on a représenté les degrés de liberté des bras en matière d'extension en longueur. Le sens transversal est en effet géré par l'aspect télescopique de chacune des poutres. En figure 12a, la vue est la même qu'en figure 11b : on a représenté l'extension des bras 20, 21 et 22, qui sont des bras rétractables ou télescopiques, dont la longueur totale peut être ajustée, indépendamment pour chaque bras.

En figure 12b, un exemple de réalisation d'un bras télescopique est représenté : il s'agit d'un système comprenant deux parties de bras, l'une constituée d'un cylindre large creusé d'un cylindre vide interne débouchant sur une section droite, et l'autre constituée d'un cylindre fin inséré dans le cylindre vide de la première partie. Les deux parties coopèrent par vissage, ou alternativement par un système de calage par encoches coopérant avec une ou plusieurs dents, les pas de vis, ou les encoches et les dents étant portées par les surfaces de contact réciproques des deux cylindres. L'amplitude du mouvement de vissage ou le choix de l'encoche ou de la dent utilisée pour le calage permet de régler la longueur du bras.

En figure 12c, un autre exemple de réalisation d'un bras télescopique est présenté : il s'agit à nouveau d'un système avec deux parties de bras, comme le précédent. Les deux parties sont immobilisées l'une par rapport à l'autre par un clapet externe qui pivote par rapport à l'une des deux parties et engage une encoche parmi une pluralité d'encoches portées par la surface extérieure de l'autre partie. Le choix de l'encoche permet de régler la longueur du bras. Alternativement, les bras ou poutres sont de longueurs fixes, et l'ajustement en longueur se fait au niveau du vérin central ou au niveau des rotules.

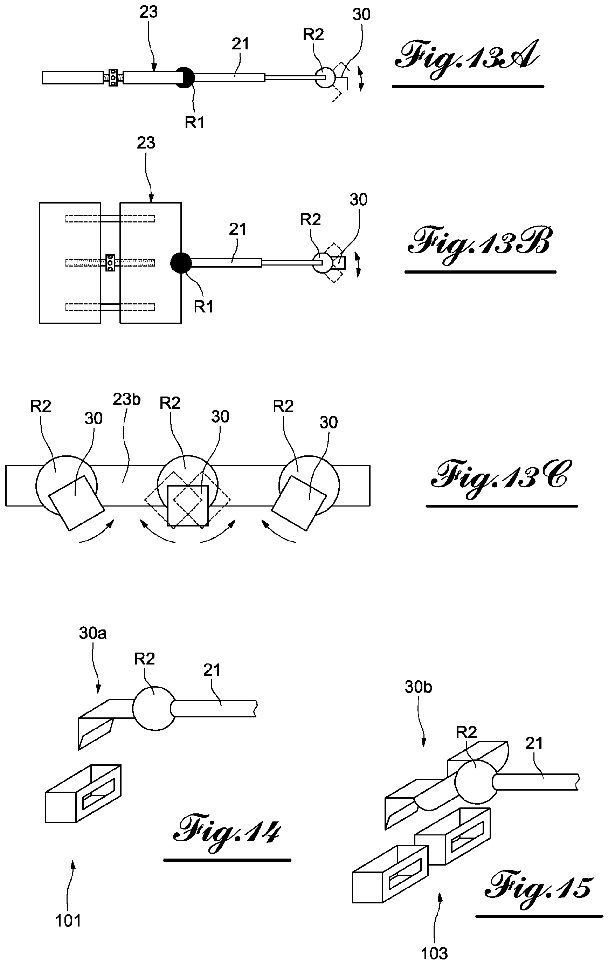

En **figure 13** **(13a à 13c),** on a représenté les degrés de liberté des bras vis-à-vis des systèmes de fixation aux dents, ou si l'on préfère, exprimé autrement, la liberté d'orientation de la tête de chaque bras vis-à-vis du bras pour permettre l'orientation des attaches qui doivent s'adapter aux dents et à leur orientation. Le bras représenté, pour l'exemple est le bras 21. Le bras 21 se termine à son extrémité liée au vérin 23 par une rotule R1 et à son autre extrémité par une rotule R2. La rotule R2 articule le bras 21 avec un élément de fixation 30 à fixer à un élément complémentaire préalablement fixé à une dent, tels ceux présentés aux figures 6 à 8.

En figures 13a et 13b, on a représenté des vues dans deux plans perpendiculaires l'un à l'autre, tous deux parallèles au bras 21. Pour simplifier l'explication, le dispositif est représenté toute chose égale par ailleurs, la rotule R1 est maintenue figée entre les deux vues, ce qui est symbolisé par le remplissage en noir sur la figure. En particulier, le vérin 23 conserve le même écartement entre les deux vues. En figure 13a, le vérin est vu de face, le plan de la figure étant perpendiculaire au plan défini par la vis 24 et les arbres 25 et 26, alors qu'en figure 13b, le vérin 23 est du haut, dans un plan parallèle à la vis 24 et aux arbres 25 et 26.

Comme on peut le voir sur les deux figures 13a et 13b, l'élément de fixation 30 peut être en rotation dans deux dimensions de l'espace vis-à-vis du bras 21, autour de deux axes perpendiculaires chacun au plan de respectivement, la figure 13a et la figure 13b.

Le troisième degré de liberté en rotation autour de la rotule R2, qui correspond à une rotation autour de l'axe du bras 21, peut être présent ou absent, même si préférentiellement, il est intéressant qu'il soit présent. Les liaisons mécaniques en rotation R2 sont ainsi choisies parmi les liaisons rotule complète, rotule à doigt, voire dans une version plus simple pivot (figure 13c).

L'ajustement au niveau des rotules R2 est très utile pour permettre l'ajustement du système. Celui-ci doit initialement être suffisamment flexible pour pouvoir être placé dans la bouche précisément à sa place finale, tout en restant passif, c'est-à-dire en n'appliquant aucune force sur les dents, les gencives et le palais. Le dispositif est ensuite activé par rotation de la vis du vérin, et entame son action à partir de la position et de la configuration dans laquelle il a été initialement installé.

La rotation au niveau de la rotule R2 peut être continue, avec une liaison mécanique, ou discrète, avec un système d'encastrement par polygone ou polyèdre, comme évoqué avec la rotation R1.

En **figure 14****,** on a représenté un élément de fixation tel qu'introduit en relation à la figure précédente, à savoir l'élément de fixation 30 placé à l'extrémité d'un bras opposée au vérin. Il est ici noté élément de fixation 30a. Un bras, par exemple le bras 21, est visible, ainsi que la rotule R2 associée. L'élément de fixation à la dent est une attache simple encoche externe 101. Elle interagit, par encastrement de type clipsage, avec une forme en crochet de l'élément de fixation 30. L'élément de fixation 30 est porté par la rotule R2 et en rotation grâce à elle, avec le bras 21.

La **figure 15** présente un autre élément de fixation tel qu'introduit en relation à la figure précédente, à savoir l'élément de fixation 30 placé à l'extrémité d'un bras opposée au vérin. Il est ici noté élément de fixation 30b. Un bras, par exemple le bras 21, est visible, ainsi que la rotule R2 associée. L'élément de fixation à la dent est une attache double encoche externe 103. Elle interagit, par encastrement de type clipsage, avec une forme en double crochet de l'élément de fixation 30b, pour augmenter la stabilité. L'élément de fixation 30b est porté par la rotule R2 et en rotation grâce à elle, avec le bras 21.

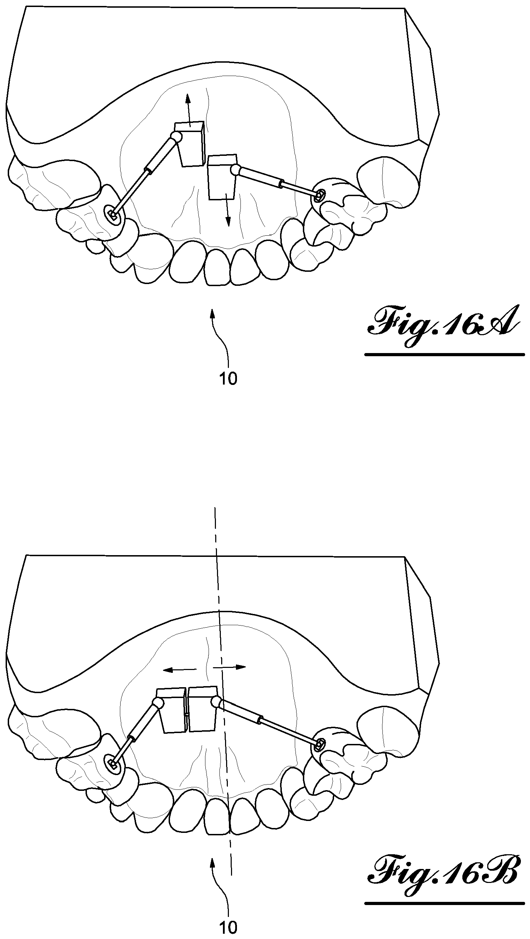

Les **figures 16a** à **16c** présentent les modes d'ajustement du système d'expansion palatine 10 dans la bouche d'un patient.

La figure 16a présente le réglage antéro-postérieur de la base, qui peut être déplacée du fond de la bouche vers l'avant de la bouche, le long de la voute palatine, en restant dans le plan horizontal (pour la discussion, on précise que quelle que soit la position du patient le plan horizontal est le plan horizontal anatomique).

La figure 16b présente le réglage transversal de la base, qui peut être déplacée du centre du palais vers la gauche du palais ou vers la droite du palais.

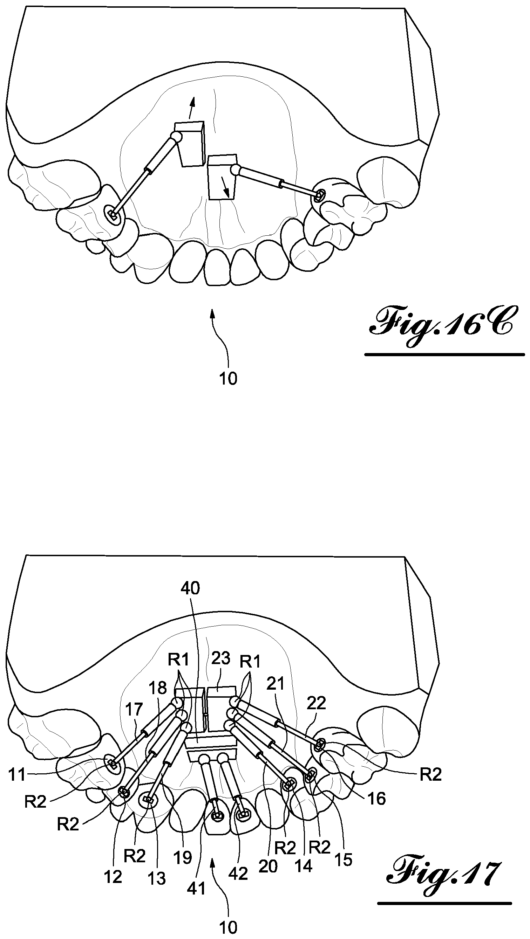

La figure 16c présente le réglage en hauteur de la base, qui peut être déplacée vers le haut (c'est-à-dire dans le cadre d'un ajustement vertical ou sagittal, ou réglage vertical de la distance de la base de l'appareil avec la voûte palatine), c'est-à-dire en direction de la voute du palais ou vers le bas, c'est-à-dire vers le centre de la bouche (le patient se tenant debout).

La **figure 17** présente un mode de réalisation du système d'expansion palatine 10a offrant un mode d'action supplémentaire qui permet une action sur d'autres sutures palatines. Il incorpore, en plus des éléments décrits ci-dessus, une deuxième base comportant une deuxième vis assurant une deuxième fonction de vérin, pour agir par exemple sur les sutures incisives du palais. On a représenté ici le vérin 23 et les bras 17 à 22. A ces éléments s'ajoute le vérin 40, dont la direction d'expansion est approximativement perpendiculaire à la direction d'expansion du vérin 23. Ce vérin 40 fournit une force d'écartement réglage entre le vérin 23 et les incisives. Deux bras 41 et 42 sont représentés entre le vérin 40 et les incisives. Des rotules peuvent avantageusement être présentes à l'une ou l'autre des extrémités de ces bras, ou aux deux extrémités. Une vis du vérin 40 assure l'écartement entre deux bras 41 et 42 et le vérin 23, placés à leur opposé par rapport au vérin 40.

Cette variante permet d'agir pour élargir le palais dans des directions différentes, en utilisant différents mouvements, pour agir sur d'autres sutures osseuses que la suture centrale du plan sagittal. D'autres dispositions utilisant différents mouvements sont envisageables, en fonction des besoins du patient, pour résoudre ses pathologies.

Le dispositif peut aussi inclure ou être utilisé avec des moyens d'appui osseux. On utilise alors des dispositifs d'appui spécifiques à visser à travers la muqueuse gingivale dans l'os du palais, par un module supplémentaire permettant la jonction avec des mini-vis d'ancrage ou des plaques d'ostéosynthèse (ajustées sur la gencive), ou encore des implants d'ancrage osseux.

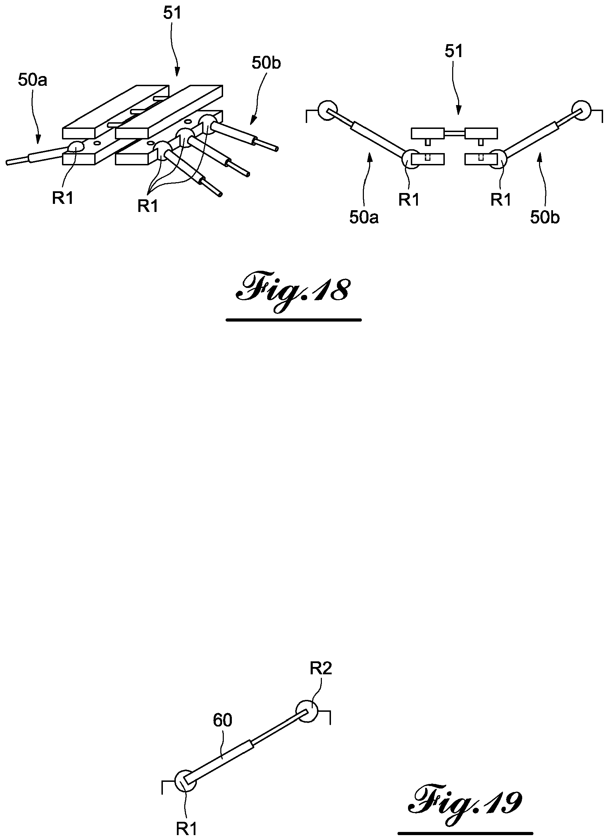

En **figure 18****,** on a représenté un mode de réalisation particulier de la base, dans laquelle trois éléments séparés sont fixés les uns aux autres pour faciliter la fabrication ou l'installation. Le système d'expansion palatine 10b comprend deux ensembles de trois bras 50a et 50b, les trois bras de chaque ensemble étant reliés entre eux par un socle commun 51 auxquels ils sont articulés par les rotules R1. Un vérin 51 indépendant est fixé par encastrement ou tout autre moyen aux deux ensembles de trois bras 50a et 50b, assurant par conséquent leur liaison. Les éléments d'expansion contrôlée du vérin (vis et arbres par exemple) sont placés de telle sorte à permettre le contrôle de la distance entre l'ensemble de trois bras 50a et l'ensemble de trois bras 50b.

En **figure 19****,** on a représenté un mode de réalisation particulier d'un bras pour le système d'expansion palatine selon l'invention. Ce bras 60 dispose à chaque extrémité d'une rotule R1 ou R2, mais est amovible par rapport à la base et au vérin. Il peut être fixé à la base à l'aide d'un élément de fixation par clipsage porté par la rotule et en rotation vis-à-vis du bras ou tout autre moyen porté par la rotule et en rotation vis-à-vis du bras, interagissant avec un moyen complémentaire porté fixe par la base. Alternativement, dans le cas d'un tel bras amovible, la rotule R1, au lieu d'être portée par le bras, peut être portée par la base, l'élément de fixation par clipsage de la base étant porté par la rotule et interagissant avec un élément complémentaire porté fixe par le bras. Dans tous les cas, le clipsage implique une forme de verrouillage.

Le dispositif peut être utilisé en orthodontie ou orthopédie dento-faciale humaine, infantile, adolescente ou adulte, et éventuellement dans une utilisation similaire pour l'animal.

La mise en place du système comprend le collage des attaches sur les dents, le positionnement de la base dans le palais, le clipsage des poutres, l'ajustement de la position et le verrouillage des rotules.

L'invention n'est pas limitée aux modes de réalisation présentés, mais s'étend à toutes les variantes dans le cadre de la portée des revendications également dans des dispositifs amovibles (pouvant être retirés par le patient) et non collés ou des dispositifs fixes, scellés ou collés (ne pouvant être retiré que par le praticien).

On précise que les mécanismes et éléments du kit présenté peuvent être associés à des éléments fabriqués par CFAO (conception et fabrication assistée par ordinateur).

L'ensemble du dispositif peut être fourni en kit c'est-à-dire un ensemble d'éléments préfabriqués qui permet de mettre en place un dispositif de disjonction ou distraction maxillaire orthodontique totalement adapté et ajusté au patient.

Il est prévu qu'à ces dispositifs soient adjointes des plaques ou gouttières (plaques avec fente) fabriquées par CFAO entrant dans une chaîne 3D de l'acquisition numérique de la cavité orale à l'impression par imprimante 3D de résine, matériau composite ou métal par procédé d'addition ou de soustraction (fraisage). Le dispositif peut ainsi être mis en place avec des éléments préfabriqués par CFAO ou autre méthode comme des plaques ou gouttières ajustées à la morphologie dentaire et/ou palatine.

Dans le cas d'utilisation de plaques ou gouttières, le dispositif est, pour le patient, amovible facilement. Sinon, il est fixe, dans le sens où seul le praticien spécialiste est en mesure de le retirer.

Des mini-vis et des implants d'ancrage orthodontique pourront aussi être associés au dispositif.

Dans une des configurations du système, il est possible de faire les réglages hors bouche, et même hors fauteuil. Une empreinte classique (avec un matériau de type alginate ou silicone) ou une empreinte optique (numérique) permet de connaître les réglages nécessaires, et l'ajustement peut se faire à l'avance sans la nécessité immédiate d'avoir le patient en présence. Un dispositif interne ou externe est de plus prévu afin de faire les réglages et l'ajustement nécessaire en avance.

Enfin, alors que l'appareil est prévu pour l'expansion palatine, il peut aussi élargir les sutures mandibulaires réelles ou artificielles (créées dans ce cas par chirurgie). Les fonctions décrites pour cet appareil lui permettent d'effectuer également des déplacements dentaires unitaires ou multiples, simultanés ou séquencés.

## Revendications

1. Dispositif d'orthodontie (10) pour expansion palatine comprenant au moins un premier moyen d'appui (17-19) pour appuyer sur la gauche d'une mâchoire d'un patient à soigner, au moins un deuxième moyen d'appui (20-22) pour appuyer sur la droite de ladite mâchoire et un vérin central (23, 51) pour moduler un écartement desdits moyens d'appui, **caractérisé en ce que** le premier ou le deuxième moyen d'appui (17-22) comprenant au moins une première poutre de liaison à mettre en place entre le vérin et une première dent, et une deuxième poutre de liaison à mettre en place entre le vérin et une deuxième dent, le dispositif (10) comprend de plus un moyen d'ajustement angulaire (R1, R2) entre le vérin et ledit premier moyen d'appui et un moyen d'ajustement angulaire (R1, R2) entre le vérin et ledit deuxième moyen d'appui pour permettre un ajustement géométrique pendant une mise en place du dispositif (10) dans la bouche du patient, au moins dans le sens vertical, le sens antéro-postérieur ou le sens transversal.

2. Dispositif d'orthodontie selon la revendication 1, dans lequel au moins une des poutres (17-22) est télescopique pour permettre un ajustement géométrique pendant une mise en place du dispositif (10) dans la bouche du patient.

3. Dispositif d'orthodontie selon la revendication 1, dans lequel au moins une des poutres (17-22) est démontable, le dispositif (10) comprenant au moins une poutre de remplacement d'une longueur différente pour permettre un ajustement géométrique pendant une mise en place du dispositif (10) dans la bouche du patient.

4. Dispositif d'orthodontie selon l'une des revendications 1 à 3, comprenant une liaison mécanique en rotation (R1) à l'extrémité de chaque poutre (17-22) entre la poutre et le vérin (23).

5. Dispositif d'orthodontie selon l'une des revendications 1 à 4, comprenant une liaison mécanique en rotation (R2) à l'extrémité de chaque poutre (17-22) opposée au vérin (23), à interposer entre la poutre et la mâchoire.

6. Dispositif d'orthodontie selon l'une des revendications 1 à 5, comprenant des attaches (100-103) de différentes dimensions et formes pour l'adaptation à différents types et morphologies de dents, à coller sur les dents, par scellement ou collage ou rétention mécanique ou chimique, et portant des moyens de liaison pour relier les attaches au vérin, le dispositif (10) comprenant de plus des éléments complémentaires de liaison reliés au vérin (23) pour mettre en place le dispositif (10) dans la bouche du patient à l'aide des attaches.

7. Dispositif d'orthodontie selon la revendication 6, dont les attaches (100-103) portent des encoches simples externes, des doubles encoches externes, des doubles encoches internes externes ou une boule externe pour une liaison par emboîtement, ou des moyens de vissage pour une liaison par vissage.

8. Dispositif d'orthodontie selon l'une des revendications 1 à 7, dans lequel le vérin (51) est fixé sur un support gauche (50a) et un support droit (50b) libres l'un par rapport à l'autre.

## Patentansprüche

1. Kieferorthopädievorrichtung (10) zur Gaumendehnung, umfassend mindestens ein erstes Stützmittel (17 - 19), um auf der linken Seite eines Kiefers eines zu behandelnden Patienten zu stützen, mindestens ein zweites Stützmittel (20 - 22), um auf der rechten Seite des Kiefers zu stützen, und einen zentralen Zylinder (23, 51), um eine Beabstandung der Stützmittel zu modulieren, **dadurch gekennzeichnet, dass** das erste oder das zweite Stützmittel (17 - 22) mindestens einen ersten Verbindungsträger, um ihn zwischen dem Zylinder und einem ersten Zahn anzuordnen, und einen zweiten Verbindungsträger, um ihn zwischen dem Zylinder und einem zweiten Zahn anzuordnen, umfasst, wobei die Vorrichtung (10) außerdem ein eckiges Einstellmittel (R1, R2) zwischen dem Zylinder und dem ersten Stützmittel und ein eckiges Einstellmittel (R1, R2) zwischen dem Zylinder und dem zweiten Stützmittel umfasst, um eine geometrische Einstellung während einer Anordnung der Vorrichtung (10) im Mund des Patienten mindestens in der vertikalen Richtung, der Richtung von vorne nach hinten oder der Querrichtung zu ermöglichen.

2. Kieferorthopädievorrichtung nach Anspruch 1, wobei mindestens einer der Träger (17 - 22) teleskopisch ist, um eine geometrische Einstellung während einer Anordnung der Vorrichtung (10) im Mund des Patienten zu ermöglichen.

3. Kieferorthopädievorrichtung nach Anspruch 1, wobei mindestens einer der Träger (17 - 22) abmontierbar ist, wobei die Vorrichtung (10) mindestens einen Ersatzträger mit einer verschiedenen Länge umfasst, um eine geometrische Einstellung während einer Anordnung der Vorrichtung (10) im Mund des Patienten zu ermöglichen.

4. Kieferorthopädievorrichtung nach einem der Ansprüche 1 bis 3, umfassend eine mechanische Verbindung in Rotation (R1) am Ende jedes Trägers (17 - 22) zwischen dem Träger und dem Zylinder (23).

5. Kieferorthopädievorrichtung nach einem der Ansprüche 1 bis 4, umfassend eine mechanische Verbindung in Rotation (R2) am Ende jedes Trägers (17 - 22) gegenüber dem Zylinder (23), um sie zwischen den Träger und den Kiefer zu stellen.

6. Kieferorthopädievorrichtung nach einem der Ansprüche 1 bis 5, umfassend Befestigungen (100 - 103) mit verschiedenen Abmessungen und Formen für die Anpassung an verschiedene Arten und Morphologien von Zähnen, um auf die Zähne durch Dichtung oder Haftung oder mechanischen oder chemischen Rückhalt zu kleben, und umfassend Verbindungsmittel, um die Befestigungen mit dem Zylinder zu verbinden, wobei die Vorrichtung (10) außerdem zusätzliche Verbindungselemente umfasst, die mit dem Zylinder (23) verbunden sind, um die Vorrichtung (10) im Mund des Patienten mit Hilfe der Befestigungen anzuordnen.

7. Kieferorthopädievorrichtung nach Anspruch 6, wobei die Befestigungen (100 - 103) einfache äußere Nuten, doppelte äußere Nuten, doppelte äußere innere Nuten oder eine äußere Kugel für eine Verbindung durch Einführen oder Mittel zum Verschrauben durch eine Schraubverbindung umfassen.

8. Kieferorthopädievorrichtung nach einem der Ansprüche 1 bis 7, wobei der Zylinder (51) auf einen linken Träger (50a) und einen rechten Träger (50b) fixiert ist, die mit Bezug aufeinander frei sind.

## Claims

1. An orthodontic device (10) for palatal expansion comprising at least one first bearing means (17-19) for bearing on the left side of a jaw of a patient to be treated, at least one second bearing means (20-22) for bearing on the right side of said jaw and a central jack (23, 51) for modulating a separation of said bearing means, **characterized in that** the first or second bearing means (17-22) comprising at least one first connecting beam to be placed between the jack and a first tooth, and a second connecting beam to be placed between the jack and a second tooth, the device (10) also comprises an angular adjusting means (R1, R2) between the jack and said first bearing means and an angular adjusting means (R1, R2) between the jack and said second bearing means to allow a geometric adjustment during a placement of the device (10) in the mouth of the patient, at least in the vertical direction, the anterior-posterior direction or the transverse direction.

2. The orthodontic device according to claim 1, wherein at least one of the beams (17-22) is telescoping to allow a geometric adjustment during a placement of the device (10) in the mouth of the patient.

3. The orthodontic device according to claim 1, wherein at least one of the beams (17-22) is removable, the device (10) comprising at least one replacement beam with a different length to allow a geometric adjustment during a placement phase of the device (10) in the mouth of the patient.

4. The orthodontic device according to one of claims 1 to 3, comprising a mechanical rotational link (R1) at the end of each beam (17-22) between the beam and the jack (23).

5. The orthodontic device according to one of claims 1 to 4, comprising a mechanical rotational link (R2) at the end of each beam (17-22) opposite the jack (23), to be inserted between the beam and the jaw.

6. The orthodontic device according to one of claims 1 to 5, comprising fasteners (100-103) with different sizes and shapes for the adaptation to different types and morphologies of teeth, to be glued on the teeth, by sealing or gluing or mechanical or chemical retention, and bearing connecting means to couple the fasteners to the jack, the device (10) also comprising complementary connecting elements coupled to the jack (23) in order to place the device (10) in the mouth of the patient using fasteners.

7. The orthodontic device according to claim 6, the fasteners (100-103) of which bear simple outer notches, double outer notches, double inner outer notches or an outer ball for a connection by interlocking, or screwing means for a connection by screwing.

8. The orthodontic device according to one of claims 1 to 7, wherein the jack (51) is fastened on a left support (50a) and a right support (50b) that are free relative to one another.
